# EUROPÄISCHE PATENTANMELDUNG

(11) **EP 2 151 489 A2**
(43) Veröffentlichungstag der Anmeldung: **10.02.2010**
(21) Anmeldenummer: 09166219.7
(22) Anmeldetag: 23.07.2009
(51) Int. Cl.: C11D 1/83, C11D 1/94, C11D 3/37, A61K 8/81, A61Q 5/00

(54) **Reinigungsmittel mit Terpolymer**

(30) Priorität: 04.08.2008 DE 102008036073
(71) Anmelder: Henkel AG & Co. KGaA, 40589 Düsseldorf (DE)
(72) Erfinder: Scheunemann, Volker, 21339 Lüneburg (DE); Schulze Zur Wiesche, Erik, 20144 Hamburg (DE)

(57) **Zusammenfassung**

Es werden tensidhaltige Reinigungsmittel auf der Basis eines kationischen Terpolymers beschrieben, die als kosmetisches Reinigungsmittel einen pH-Wert im Bereich von 2 bis 5,5, und als Reinigungsmittel für harte Oberflächen und/oder Textilien einen pH-Wert im Bereich von 2 bis 11 aufweisen.

Die kosmetischen Mittel dieser Erfindung eignen sich hervorragend zur Verbesserung der sensorischen Eigenschaften der Haare sowie zur innerstrukturellen Stabilisierung der Haare.

## Beschreibung

Die Erfindung betrifft tensidhaltige Reinigungsmittel auf der Basis eines kationischen Terpolymeren, die -je nach Anwendungsform - einen bestimmten pH-Bereich aufweisen, die Verwendung des kosmetischen Mittels zur Verbesserung der sensorischen Eigenschaften der Haare und zur innerstrukturellen Stabilisierung der Haare sowie ein Verfahren zur Haarbehandlung unter Verwendung des kosmetischen Mittels.

Bedingt durch das stetig steigende Hygiene-Empfinden der Menschen und die damit immer größer und breiter werdenden Anforderungen an Reinigungsmittel, insbesondere an kosmetische Produkte, ergeben sich fortwährend neue Problemstellungen und Schwierigkeiten bei deren Herstellung. Um den Bedürfnissen der Verbraucher gerecht zu werden, müssen Reinigungsmittel, insbesondere kosmetische Reinigungsmittel für Haut und Haare, wie sie beispielsweise als flüssige Seifen, Shampoos, Duschbäder, Schaumbäder, Dusch- und Waschgele im Handel erhältlich sind, heutzutage nicht nur ein gutes Reinigungsvermögen aufweisen, sondern sollen weiterhin gut verträglich sein und auch bei häufiger Anwendung nicht zu starker Entfettung oder Trockenheit der Haut oder der Haare führen.

Diesen Ansprüchen kann durch die gezielte Wahl geeigneter Wirkstoffe entsprochen werden, doch gerade die Kombination einer Vielzahl unterschiedlicher Wirkstoffe kann unerwünschte Wechselwirkungen unter der Wirkstoffen hervorrufen, oder es kann eine unerwünschte pH-Wert-Verschiebung stattfinden, wodurch die Wirksamkeit einiger Komponenten nachlässt oder gar verloren geht.

Übliche (kosmetische) Reinigungsprodukte enthalten Tenside in Mengen von etwa 10 bis 15 Gew.-%, um eine zufriedenstellende Reinigungsleistung zu erzielen. Nach der Anwendung eines solchen Reinigungsprodukts empfindet der Verbraucher seinen Hautzustand jedoch oftmals als nicht ideal, was vorwiegend auf die leicht irritierende Wirkung der meisten Tenside zurückzuführen ist. Der Hautzustand nach der Anwendung wird als trocken, gespannt und mitunter als rau empfunden, weshalb die nachträgliche Applikation von Cremes oder Lotionen erforderlich wird. Dies hat zum einen den Nachteil eines höheren Zeitaufwands und zum anderen den Nachteil eines höheren Kostenaufwands, da mindestens zwei Produkte benötigt werden.

In der letzten Zeit hat es daher nicht an Versuchen gefehlt Mittel zur Verfügung zu stellen, die nicht nur eine zufriedenstellende Reinigung von harten Oberflächen, Textilien, Haut und/oder Haaren, sondern auch spürbare Pflegeeigenschaften auf Textilien, der Haut oder der Haare aufweisen. So wurden diverse Fettstoffe, Silikone und/oder kationische Polymere, kationische Tenside, spezielle Pflanzenextrakte etc. eingesetzt, um die gewünschte Wirkung zu erzielen.

Ziel der vorliegenden Erfindung war es daher maschinelle und manuelle Reinigungsmittel für harte Oberflächen, Textilien und kosmetische Zwecke, insbesondere ein Haut- und Haarreinigungsmittel zur Verfügung zu stellen, die in jeder Phase der Anwendung spürbare Pflegeeigenschaften auf der jeweiligen Oberfläche aufweisen. Daneben soll die Schaumqualität der Mittel und deren Stabilität - insbesondere die Stabilität der Viskosität - verbessert werden. Insbesondere sollen die kosmetischen Reinigungsmittel die sensorischen Eigenschaften der Haare wie Griff und Weichheit verbessern. Daneben soll aber auch eine Splissreduktion sowie eine verbesserte Naß- und Trockenkämmbarkeit der Haare erreicht werden.

Schließlich ist es auch Ziel der Erfindung nicht nur die äußere Struktur der Haare zu verbessern, sondern auch eine innerstrukturelle, stabilisierende Wirkung auf den Haaren zu erreichen.

Bei nicht-kosmetischen Reinigungsmitteln soll die Oberfläche gründlich von Flecken, Schmutz und Ablagerungen gereinigt werden, ohne dass unerwünschte Rückstände, Wasserflecken oder Streifen zurückbleiben. Die Textilien sollen sich zudem nach der Reinigung gepflegt und weich anfühlen und die Haut soll sich nach der Anwendung der manuellen Reinigungsmittel weniger rau und ausgetrocknet anfühlen.

Vollkommen überraschend wurde nun gefunden, dass die Inkorporation eines speziellen Terpolymers in maschinelle, manuelle und kosmetische Reinigungsmittel die oben genannten Anforderungen in hohem Maße erfüllen.

Die Verwendung der Terpolymere in kosmetischen Haarreinigungsmitteln ist bekannt, doch betreffen diese Reinigungsmittel ausschließlich Produkte mit einem pH-Wert von >6,2.

Für die kosmetischen Mittel der vorliegenden Erfindung werden zur Erfüllung der o.g. Anforderungen aber niedrigere pH-Werte bis zu einem maximalen pH-Wert von 5,5 angestrebt. Die Verwendung der Terpolymere in Reinigungsmitteln für harte Oberflächen und/oder Textilien ist hingegen nicht bekannt.

Gegenstand der vorliegenden Erfindung sind damit tensidhaltige Reinigungsmittel enthaltend - bezogen auf ihr Gesamtgewicht - 0,001 bis 10 Gew.-% mindestens eines kationischen Terpolymers, das zusammengesetzt ist aus
a) mindestens einem Vinylimidazol-Monomeren,
b) mindestens einem Vinyl-Pyrrolidon-Monomeren und
c) Poly(dimethyldiallylammoniumchlorid),
**dadurch gekennzeichnet, dass** das Mittel einen pH-Wert von 2 bis 5,5 aufweist, wenn es als kosmetisches Reinigungsmittel dient, und einen pH-Wert im Bereich von 2 bis 11, wenn es als Reinigungsmittel für harte Oberflächen und/oder Textilien formuliert wird.

Es wurde gefunden, dass die Terpolymere den erfindungsgemäßen Mitteln eine verbesserte Schaumqualität verleihen und viskositätsstabilisierend wirken. Dieser Effekt macht sich vor allen Dingen bei höheren Temperaturen bemerkbar.

Weiterhin konnten erfindungsgemäße Mittel, die als kosmetische Mittel konfektioniert wurden, die Kämmbarkeit und den Glanz der damit behandelten Haar erhöhen sowie die statische Aufladung der Haare beim Kämmen verringern, und Haarschädigungen wie Haarbruch und Spliss lindern. Bevorzugte erfindungsgemäße Mittel enthalten Terpolymere mit einer kationischen Ladungsdichte von 2,5 bis 5 meq/g, bevorzugt 3 bis 4,5 meq/g und insbesondere 3,5 bis 4 meq/g (bei pH 7). Das Molekulargewicht der Terpolymere in den erfindungsgemäßen Reinigungsmitteln beträgt 50,000 bis 500,000, bevorzugt 75,000 bis 300,000 und insbesondere 100,000 bis 200,000.

Das Terpolymer wird in einer Menge von 0,005 bis 5 Gew.-%, bevorzugt in einer Menge von 0,01 bis 4 Gew.-% und insbesondere bevorzugt in einer Menge von 0,05 bis 3 Gew.-% - bezogen auf das Gesamtgewicht des Reinigungsmittels, eingesetzt.

Aus formulierungstechnischen Gründen kann es bevorzugt sein, dass das erfindungsgemäße Terpolymer als wässrige Lösung in die erfindungsgemäßen Reinigungsmittel eingearbeitet wird. Ein erfindungsgemäß besonders bevorzugtes Terpolymer ist das unter der INCI-Bezeichnung "Polyquaternium-87" bekannte, beispielsweise von der Firma BASF unter dem Handelsnamen "Luviquat Sensation" vertriebene Terpolymer. Dieses liegt als 26%ige Lösung in Wasser vor.

Alle erfindungsgemäßen Reinigungsmittel enthalten zur Erzielung einer Reinigungsleistung Tenside, die üblichweise ausgewählt sein können aus anionischen, amphoteren/zwitterionischen, nichtionischen und/oder kationischen Tensiden.

Da anionische Tenside sich erfindungsgemäß als unbedingt erforderlich erwiesen haben, damit eine zufriedenstellende Reinigungsleistung erzielt wird, da aber gleichzeitig eine gewisse Milde der Tenside gewährleistet werden soll, hat es sich als erfindungsgemäß bevorzugt herausgestellt, wenn die erfindungsgemäßen Reinigungsmittel mindestens ein anionisches Tensid enthalten und weiterhin mindestens ein amphoteres/zwitterionisches und/oder nichtionisches Tensid.

Bei den erfindungsgemäßen kosmetischen Mitteln hat es sich zudem als besonders vorteilhaft erwiesen, wenn als Reinigungstensid ein Gemisch aus anionischen Tensiden mit amphoteren/zwitterionischen und/oder nichtionischen Tensiden eingesetzt wird, bei denen das Gewichtsverhältnis des anionischen Tensids zu den amphoteren/zwitterionischen und nichtionischen Tensiden (1-2) : (0,5-1) : (0,5-1) beträgt.

Der Gesamttensidgehalt in den Reinigungsmitteln beträgt 3 bis 50 Gew.-%, bevorzugt 5 bis 40 Gew.-% und insbesondere 7 bis 25 Gew.-% - bezogen auf das Gesamtgewicht des Reinigungsmittels.

Als anionische Tenside eignen sich in erfindungsgemäßen Zubereitungen alle anionischen Tenside; für die Verwendung am menschlichen Körper eignen sich insbesondere für die kosmetische Verwendung geeignete anionische oberflächenaktive Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)_{X}-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓOSO₃H, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (II), 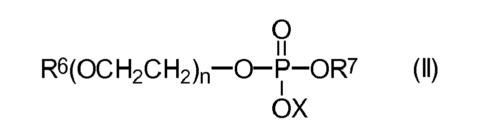 in der R⁶ bevorzugt für einen aliphatischen Kohlenwasserstoffrest mit 8 bis 30 C-atomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁ bis C₄ - Kohlenwasserstoffrest, steht,
- sulfatierte Fettsäurealkylenglykolester der Formel (III),

   R¹²CO(AlkO)ₙSO₃M (III)

   in der R¹²CO- für einen linearen oder verzweigten, aliphatischen, gesättigten und/oder ungesättigten Acylrest mit 6 bis 22 C-Atomen, Alk für Ethyl-, Propyl und/oder Isopropyl-, n für Zahlen von 0,5 bis 5 und M für ein Kation steht, Monoglyceridsulfate und Monoglyceridethersulfate der Formel (IV), 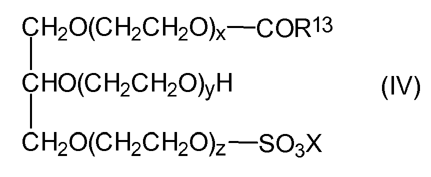 in der R¹³CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Vorzugsweise werden Monoglyceridsulfate der Formel (VIII) eingesetzt, in der R¹³CO für einen linearen Acylrest mit 8 bis 18 Kohlenstoffatomen steht.

Bevorzugte anionische Tenside für alle erfindungsgemäßen Reinigungsmittel sind Alkylsulfate, Alkylpolyglykolethersulfate mit linearen oder verzweigten C-Ketten und Ethercarbonsäuresalze mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe, Sulfobernsteinsäuremono-alkylpolyoxyethylester mit 8 bis 18 C-Atomen in der Alkylgruppe und 1 bis 6 Oxyethylgruppen sowie die Alkali- und/oder Ammoniumsalze des Tridecethsulfats.

Besonders bevorzugte anionische Tenside sind die Alkali- oder Ammoniumsalze des Laurylethersulfates mit einem Ethoxylierungsgrad von 2 bis 4 EO.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-C₆- Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl, beispielsweise Rizinusöl-hydriert+40 EO, wie es beispielsweise unter dem Handelsnamen Cremophor CO 455 von der Firma SHC im Handel erhältlich ist,
- Polyolfettsäureester, wie beispielsweise Hydagen^{®} HSP (Cognis) oder Sovermol (Cognis),
- alkoxilierte Triglyceride,
- alkoxilierte Fettsäurealkylester der Formel (V)

   R¹⁴CO-(OCH₂CHR¹⁵)_{w}OR¹⁶ (V)

   in der R¹⁴CO für einen linearen oder verzweigten, gesättigten und/oder ungesättigten Acylrest mit 6 bis 22 Kohlenstoffatomen, R¹⁵ für Wasserstoff oder Methyl, R¹⁶ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als R Mischungen entsprechend den Ausgangsverbindungen vor.

Besonders bevorzugt sind solche Alkylpolyglykoside, bei denen R im wesentlichen aus C₈ und C₁₀-Alkylgruppen, im wesentlichen aus C₁₂- und C₁₄-Alkylgruppen, im wesentlichen aus C₈- bis C₁₆-Alkylgruppen, im wesentlichen aus C₁₂- bis C₁₆-Alkylgruppen oder im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 C-atome auf. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer besonders bevorzugten Ausführungsform der Erfindung wird das erfindungsgemäße Reinigungsmittel als kosmetisches Reinigungsmittel konfektioniert. In diesem Fall enthält es weiterhin Panthenol sowie mindestens einen weiteren Haarpflegewirkstoff, der ausgewählt ist aus der Gruppe der Vitamine und/oder Provitamine, der Proteinhydrolysate, der natürlichen oder synthetischen Ölkomponenten und/oder der Pflanzenextrakte.

Unter erfindungsgemäß bevorzugten Vitaminen, Provitaminen und Vitaminvorstufen sowie deren Derivaten sind solche Vertreter zu verstehen, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,01-1 Gew.-%, bezogen auf die gesamte Zubereitung.

Zur Vitamin B-Gruppe oder zu dem Vitamin B-Komplex gehören u. a.

Vitamin B₁ (Thiamin)

Vitamin B₂ (Riboflavin)

Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäßen Mitteln bevorzugt in Mengen von 0,05 bis 1 Gew.-%, bezogen auf das gesamte Mittel, enthalten ist.

Vitamin B₅ (Pantothensäure, Panthenol und Pantolacton). Im Rahmen dieser Gruppe wird bevorzugt Pantolacton eingesetzt.

Vitamin B₆ (Pyridoxin sowie Pyridoxamin und Pyridoxal).

Die genannten Verbindungen der Vitamin B-Gruppe sind in den erfindungsgemäßen Mitteln bevorzugt in Mengen von 0,01 - 2 Gew.-%, bezogen auf das gesamte Mittel, enthalten. Mengen von 0,03 - 1 Gew.-% sind besonders bevorzugt.

Vitamin C (Ascorbinsäure). Vitamin C wird in den erfindungsgemäßen Mitteln bevorzugt in Mengen von 0,01 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,01-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*,6a*R*)-2-Oxohexahydrothienol[3,4-*d*]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zubereitungen Vitamine, Provitamine und Vitaminvorstufen aus den Gruppen A, E, F und H. Selbstverständlich können auch mehrere Vitamine und Vitaminvorstufen gleichzeitig enthalten sein.

Die Gesamt-Einsatzmenge der Vitamine, Provitamine, Vitaminvorstufen sowie deren Derivate in den erfindungsgemäßen Mitteln beträgt - bezogen auf das Gesamtgewicht des Mittels - 0,01 bis 5 Gew.-%, vorzugsweise 0,02 bis 4 Gew.-% und insbesondere 0,05 bis 3 Gew.-%.

Als Proteinhydrolysate im Sinne der Erfindung werden Proteinhydrolysate und/oder Aminosäuren und deren Derivate (H) verstanden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgeweicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden.

Die Proteinhydrolysate oder deren Derivate sind in den erfindungsgemäßen Zubereitungen bevorzugt in Mengen von 0,1 bis 10 Gew.-%, bezogen auf das gesamte Mittel, enthalten. Mengen von 0,1 bis 5 Gew.-% sind besonders bevorzugt.

Als wasserunlösliche Ölkomponenten eignen sich erfindungsgemäß pflanzliche, mineralische oder synthetische Öle, sowie Gemische dieser Komponenten.

Als natürliche (pflanzliche) Öle werden üblicherweise Triglyceride und Mischungen von Triglyceriden eingesetzt. Bevorzugte natürliche Öle im Sinne der Erfindung sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Babssuöl, Olivenöl, Weizenkeimöl, Kürbiskernöl, Malvenöl, Haselnussöl, Safloröl, Canolaöl, Sasanquaöl, Jojobaöl und Shea-Butter.

Als mineralische Öle kommen insbesondere Mineralöle, Paraffin- und Isoparaffinöle sowie synthetische Kohlenwasserstoffe zum Einsatz. Ein erfindungsgemäß einsetzbarer Kohlenwasserstoff ist beispielsweise das als Handelsprodukt erhältliche 1,3-Di-(2-ethylhexyl)-cyclohexan (Cetiol^{®} S).

Als synthetische Öle kommen Silikonverbindungen, insbesondere Dialkyl- und Alkylarylsilikone, wie beispielsweise Dimethylpolysiloxan und Methylphenylpolysiloxan, sowie deren hydroxyterminierte, alkoxylierte und quaternierte Analoga in Betracht. Beispiele für solche Silikone sind die von Dow Corning unter den Bezeichnungen DC 190, DC 200, DC 344 und DC 345 (Cyclomethicone) vertriebenen Produkte.

Als Ölkomponente kann weiterhin ein Dialkylether dienen.

Erfindungsgemäß einsetzbare Dialkylether sind insbesondere Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyln-undecylether sowie Di-tert.-butylether, Di-iso-pentylether, Di-3-ethyldecylether, tert.-Butyl-n-octylether, iso-Pentyl-n-octylether und 2-Methylpentyl-n-octylether.

Erfindungsgemäß besonders bevorzugt ist der Di-n-octylether, der im Handel unter der Bezeichnung Cetiol^{®} OE erhältlich ist.

Die erfindungsgemäßen Mittel enthalten die wasserunlösliche Ölkomponente bevorzugt in einem Mengenbereich von 0,1 bis 5 Gew.-%, insbesondere von 0,5 bis 3 Gew.-%, bezogen auf das Gesamtgewicht des Mittels.

In einer weiteren bevorzugten Ausführungsform der Erfindung kann die Wirkung der erfindungsgemäßen Wirkstoffkombination durch weitere Fettstoffe noch weiter optimiert werden. Unter weiteren Fettstoffen sind zu verstehen Fettsäuren, Fettalkohole sowie natürliche und synthetische Wachse, welche sowohl in fester Form als auch flüssig in wäßriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Weitere bevorzugte erfindungsgemäße Fettalkohole sind die Substanzen, die beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben sind. Selbstverständlich können erfindungsgemäß auch Wollwachsalkohole, wie beispielsweise die Handelsprodukte Corona^{®}, White Swan^{®}, Coronet^{®} oder Fluilan^{®}, eingesetzt werden.

Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Weitere Fettstoffe sind beispielsweise
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, My ristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol- caprinat/-caprylat (Cetiol^{®} LC), n-Butylstearat, Oleylerucat (Cetiol^{®} J 600), Isopropylpalmitat Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Die Einsatzmenge der weiteren Fettstoffe beträgt 0,1 -20 Gew. % bezogen auf das gesamte Mittel. Bevorzugt sind 0,1 - 10 Gew.% und besonders bevorzugt 0,1 - 5 Gew.%, bezogen auf das gesamte Mittel.

Die Gesamtmenge an Öl- und Fettkomponenten in den erfindungsgemäßen Mitteln beträgt üblicherweise 0,01 - 15 Gew.-%, bezogen auf das gesamte Mittel. Mengen von 0,05- 5 Gew.-% sind erfindungsgemäß bevorzugt.

Erfindungsgemäß bevorzugt sind tensidhaltige Reinigungsmittel, die Pflanzenextrakte aus grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Granatapfel, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Vanille, Eibisch, Meristem, Ginseng und Ingwerwurzel enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Die Pflanzenextrakte werden in den erfindungsgemäßen Mitteln - bezogen auf deren Gewicht - in einer Menge von 0,01 bis 5 Gew.-%, vorzugsweise 0,02 bis 4 Gew.-% und insbesondere 0,05 bis 3 Gew.-% eingesetzt.

In einer weiteren bevorzugten Ausführungsform der Erfindung enthalten die erfindungsgemäßen Mittel mindestens ein weiteres Konservierungsmittel, ausgewählt aus der Gruppe, die gebildet wird aus Benzoesäure, Propionsäure, Salicylsäure und/oder Sorbinsäure, sowie aus den kosmetisch verträglichen Salzen und Estern dieser Säuren, aus Formaldehyl und/oder Paraformaldehyd, aus Benzylalkohol, Germall, Dowicil, Kathon, Baypival, Methylisothiazoline, quaternären Ammoniumverbindungen, Alkoholen und/oder aus etherischen Ölen wie Teebaumöl, Cineol, Eugenol, Geraniol, Limonen, Menthol und/oder Thymol.

Für den Fall, in dem das erfindungsgemäße kosmetische Mittel als Shampoo konfektioniert wird, kann es bevorzugt sein, dass dieses mindestens ein Antischuppenmittel in einer Menge von 0,05 bis 5 Gew.-%, bevorzugt von 0,1 bis 3,0 Gew.-% und insbesondere von 0,3 bis 2,0 Gew.-% enthält (bezogen auf das gesamte Mittel).

Diese(s) ist (sind) beispielsweise ausgewählt aus Piroctone Olamine, Climbazol, Zink Pyrithion, Ketoconazole, Salicylsäure, Schwefel, Selensulfide, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten.

In einer weiteren bevorzugten Ausführungsform der Erfindung enthält das erfindungsgemäße Mittel weiterhin mindestens einen Vertreter aus der Gruppe der kationischen Tenside und/oder Polymere, der UV-Filter, der Aminosäuren, der wasserunlöslichen Silikone, der wasserlöslichen Silikone und/oder der Amodimethicone.

Unter erfindungsgemäß geeigneten kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (VI), 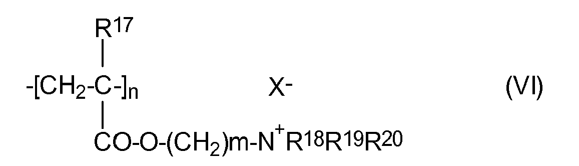 in der R¹⁷ = -H oder -CH₃ ist, R¹⁸, R¹⁹ und R²⁰ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im Wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁷ steht für eine Methylgruppe
- R¹⁸, R¹⁹ und R²⁰ stehen für Methylgruppen
- m hat den Wert 2
- X⁻ steht für Halogenid-, Sulfat-, Phosphat-, Methosulfat- sowie org. Ionen wie Lactat-, Citrat-, Tartrat- und Acetationen in Betracht. Bevorzugt sind Halogenidionen, insbesondere Chlorid.

Ein geeignetes Homopolymer ist das, gewünschtenfalls vernetzte, Poly(methacryloyloxyethyltrimethylammoniumchlorid) mit der INCI-Bezeichnung Polyquaternium-37.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (INCI-Bezeichnung: Mineral Oil) und Tridecylpolyoxypropylen-polyoxyethylen-ether (INCI-Bezeichnung: PPG-1-Trideceth-6)) und Salcare^{®} SC 96 (INCI-Bezeichnung: Propylene Glycol Dicaprylate/Dicaprate) und Tridecyl-polyoxypropylenpolyoxyethylen-ether (INCI-Bezeichnung: PPG-1-Trideceth-6)) im Handel erhältlich.

Copolymere mit Monomereinheiten gemäß Formel (VI) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer, wie sie unter der Bezeichnung Salcare^{®} SC 92 erhältlich sind.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure, wie beispielsweise die Handelsprodukte Merquat^{®}100 und Merquat^{®}550,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere, wie beispielsweise die Handelsprodukte Gafquat^{®}734 und Gafquat^{®}755,
- Vinylpyrrolidon-Vinylimidazoliummethochlorid-Copolymere, wie beispielsweise die Handelsprodukte Luviquat^{®} FC 370, FC 550, FC 905 und HM 552,
- quaternierter Polyvinylalkohol, sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

In einer besonders bevorzugten Ausführungsform der Erfindung ist als kationisches Polymer mindestens ein Polymer aus der Gruppe der kationischen Guar-Derivate und/oder Polyquaternium-7 (Merquat 550), Polyquaternium-6, Polyquaternium-10 und/oder Polyquaternium-67 (SoftCat^{®}-Polymere) in den Reinigungsmitteln enthalten.

Das oder die kationische(n) Polymer(e) ist (sind) in den erfindungsgemäßen Mitteln - bezogen auf das gesamte Mittel - in Mengen von 0,01 bis 5 Gew.-% enthalten. Mengen von 0,05 bis 3, insbesondere von 0,1 bis 2 Gew.-%, sind besonders bevorzugt.

Weiterhin kann in einer bevorzugten Ausführungsform der Erfindung die Wirkung der Zubereitungen durch UV - Filter gesteigert werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß verwendeten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern. Bevorzugt sind solche UV-Filter, deren molarer Extinktionskoeffizient am Absorptionsmaximum oberhalb von 15 000, insbesondere oberhalb von 20000, liegt.

Gemäß einer weiteren Ausführungsform der Erfindung sind solche UV-Filter bevorzugt, die eine kationische Gruppe, insbesondere eine quartäre Ammoniumgruppe, aufweisen.

Zwei bevorzugte UV-Filter mit kationischen Gruppen sind die als Handelsprodukte erhältlichen Verbindungen Zimtsäureamidopropyl-trimethylammoniumchlorid (Incroquat^{®}UV-283) und Dodecyl-dimethylaminobenzamidopropyl-dimethylammoniumtosylat (Escalol^{®} HP 610).

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt. Erfindungsgemäß geeignete Aminosäuren sind Lysin, Isoleucin, Tyrosin, Cystein, Cystin, Alanin, Serin, Tryptophan, Glutaminsäure, Threonin, Valin, Glycin, Prolin, Arginin und/oder Taurin. Sie werden in den erfindungsgemäßen Haarreinigungs- und -konditioniermitteln in einer Konzentration von 0,01 bis 5 Gew.-%, insbesondere in einer Konzentration von 0,05 bis 3 Gew.-%, eingesetzt.

Gemäß einer weiteren bevorzugten Ausführungsform der Erfindung enthalten die Mittel außerdem mindestens ein flüchtiges oder nichtflüchtiges, wasserunlösliches Silikon, wasserlösliches und/oder aminofuktionalisiertes Silikon.

Erfindungsgemäß geeignete Silikone bewirken die unterschiedlichsten Effekte. So beeinflussen sie beispielsweise gleichzeitig die Trocken- und Nasskämmbarkeiten, den Griff des trockenen und nassen Haares sowie den Glanz. Unter dem Begriff Silikone versteht der Fachmann mehrere Strukturen siliciumorganischer Verbindungen.

Dimethiconole (S1) bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

(SiOHR¹_{z}) - O - (SiR²₂ - O - )ₓ - (SiOHR¹₂) (S1 - I)

Verzweigte Dimethiconole können durch die Strukturformel (S1 - II) dargestellt werden:

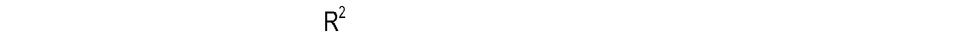

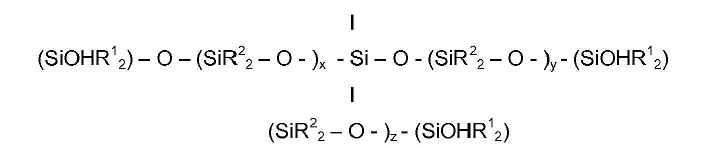

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconole bereits als Emulsion vorliegen können. Dabei kann die entsprechende Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Wenn die Dimethiconole (S1) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconol bezogen auf die Zusammensetzung.

Erfindungsgemäß besonders geeignet sind auch die sogenannten Dimethicone (S2). Diese können sowohl linear als auch verzweigt als auch cyclisch oder cyclisch und verzweigt sein. Lineare Dimethicone können durch die folgende Strukturformel (S2 - I) dargestellt werden:

(SiR¹₃) - O - (SiR²₂ - O - )x - (SiR¹₃) (S2 - I)

Verzweigte Dimethicone können durch die Strukturformel (S2 - II) dargestellt werden:

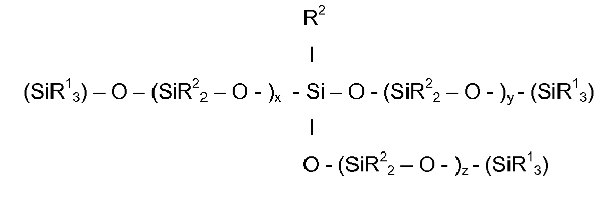

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000.

Insbesondere bevorzugt sind erfindungsgemäß die linearen Polydialkylsiloxane, die Polyalkylarylsiloxane, die Polydiarylsiloxane und/oder die Dihydroxypolydimethylsiloxane.

Die Molgewichte der erfindungsgemäß geeigneten Dimethicone liegen zwischen 1000 D und 10000000 D.

Die Viskositäten der erfindungsgemäß geeigneten Dimethicone liegen üblicherweise zwischen 0,01 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 0,1 und 5000000 cPs, ganz besonders bevorzugte Vsikositäten liegen zwischen 0,1 und 3000000 cPs. Der am meisten bevorzugte Viskositätsbereich der Dimethicone liegt zwischen 0,6 und 600000 cPs.

Selbstverständlich umfasst die erfindungsgemäße Lehre auch, dass die Dimethicone bereits als Emulsion vorliegen können. Dabei kann die entsprechende Emulsion der Dimethicone sowohl nach der Herstellung der entsprechenden Dimethicone aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethicone auch direkt durch ein Emulsionspolymerisationsverfahren hergestellt werden.

Wenn die erfindungsgemäßen Dimethicone als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethicone verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethicone ganz besonders bevorzugt sein. Wenn die Dimethicone (S2) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethicon, bezogen auf die Zusammensetzung.

Dimethiconcopolyole (S3) bilden eine weitere Gruppe bevorzugter Silikone. Dimethiconole können durch die folgende Strukturformeln dargestellt werden:

(SiR¹₃) - O - (SiR²₂ - O - )ₓ - (SiRPE - O - )_{y} - (SiR¹₃) (S3 - I)

oder durch die nachfolgende Strukturformel:

PE - (SiR¹₂) - O - (SiR²₂ - O - )ₓ -(SiR¹₂) - PE (S3 - II)

Verzweigte Dimethiconcopolyole können durch die Strukturformel (S3 - III) dargestellt werden: 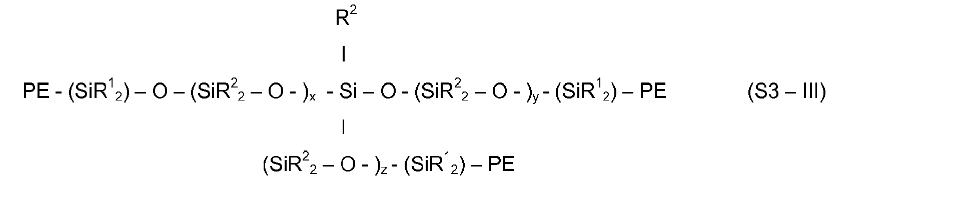 oder durch die Strukturformel(S3 - IV):

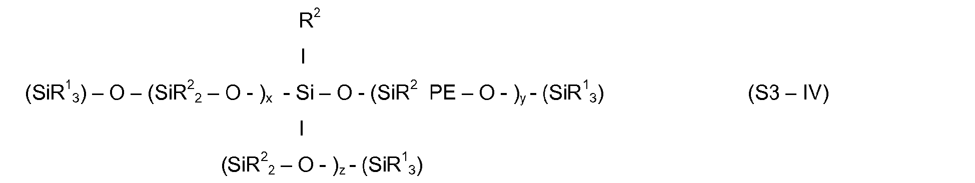

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH ₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconcopolymere bereits als Emulsion vorliegen können. Dabei kann die entsprechende Emulsion der Dimethiconcopolyole sowohl nach der Herstellung der entsprechenden Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconcopolyole auch direkt durch ein Emulsionspolymerisationsverfahren hergestellt werden.

Wenn die erfindungsgemäßen Dimethiconcopolyole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt. Werden verzweigte Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconcopolyole ganz besonders bevorzugt sein.

Wenn die Dimethiconcopolyole (S3) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Dimethiconcopolyol bezogen auf die Zusammensetzung.

Aminofunktionelle Silikone oder auch Amodimethicone (S4) genannt, sind Silicone, welche mindestens eine (gegebenenfalls substituierte) Aminogruppe aufweisen.
Solche Silicone können z.B. durch die Formel (S4 - I)

M(RₐQ_{b}SiO_{(4-a-b)/2)x}(P_{c}SiO_{(4-c)/2)y}M (S4 - I)

Beschreiben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, - CH₂CH₂OCH₂-, -OCH₂CH₂-, -OCH₂CH₂CH₂-, -CH₂CH(CH₃)C(O)OCH₂-, -(CH₂)₃CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)₂NH₂, worin z 1 oder mehr ist. Eine andere mögliche Formel für Z ist -NH(CH₂)_{z}(CH₂)_{zz}NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und z 0 ist.

Q ist am bevorzugtesten ein polarer, aminfunktioneller Rest der Formel - CH₂CH₂CH₂NHCH₂CH₂NH₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind **dadurch gekennzeichnet, daß** sie ein aminofunktionelles Silikon der Formel (S4 - II)

R'ₐG₃₋ₐ-Si(OSiG₂)ₙ-(OSiG_{b}R'_{2-b})ₘ-O-SiG₃₋ₐ-R'ₐ (S4 - II),

enthalten, worin bedeutet:
- G ist-H, eine Phenylgruppe, -OH, -O-CH₃, -CH₃, -CH₂CH₃, -CH₂CH₂CH₃, -CH(CH₃)₂, - CH₂CH₂CH₂H₃, -CH₂CH(CH₃)₂, -CH(CH₃)CH₂CH₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   o -N(R")-CH₂-CH₂- N(R")₂
   o -N(R")₂
   o -N⁺(R")₃A⁻
   o -N⁺H(R")₂A⁻
   o -N⁺H₂(R")A⁻
   o -N(R")-CH₂-CH₂-N⁺R"H₂A⁻,
      wobei jedes R" für gleiche oder verschiedene Reste aus der Gruppe -H, -Phenyl, - Benzyl, der C₁₋₂₀-Alkyl, vorzugsweise -CH₃, -CH₂CH₃, -CH₂CH₂CH₃, -CH(CH₃)₂, - CH₂CH₂CH₂H₃, -CH₂CH(CH₃)₂, -CH(CH₃)CH₂CH₃, -C(CH₃)₃, steht, und A ein Anion ist,
      das vorzugsweise ausgewählt ist aus Chlorid, Bromid, lodid oder Methosulfat. Besonders bevorzugte erfindungsgemäße Mittel sind **dadurch gekennzeichnet, daß** sie ein aminofunktionelles Silikon der Formel (S4 - III) 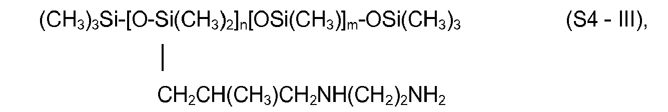
enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Trimethylsilylamodimethicone bezeichnet. Besonders bevorzugt sind auch erfindungsgemäße Mittel, die **dadurch gekennzeichnet** sind, daß sie ein aminofunktionelles Silikon der Formel (S4 - IV) 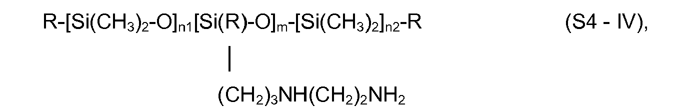 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt,
wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Wenn die Amodimethicone (S4) in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Amodimethicon bezogen auf die Zusammensetzung.

Werden unterschiedliche Silikone als Mischung verwendet, so ist das Mischungsverhältnis weitgehend variabel. Bevorzugt werden jedoch alle zur Mischung verwendeten Silikone in einem Verhältnis von 5 : 1 bis 1 : 5 im Falle einer binären Mischung verwendet. Ein Verhältnis von 3 : 1 bis 1 : 3 ist besonders bevorzugt. Ganz besonders bevorzugte Mischungen enthalten alle in der Mischung enthaltenen Silikone weitestgehend in einem Verhältnis von etwa 1 : 1, jeweils bezogen auf die eingesetzten Mengen in Gew.%.

Wenn die Silikonmischung in der Basiszusammensetzung enthalten sind, so enthalten diese Zusammensetzungen 0,01 bis 10 Gew.%, vorzugsweise 0,1 bis 8 Gew.%, besonders bevorzugt 0,25 bis 7,5 Gew.% und insbesondere 0,5 bis 5 Gew.% an Silikonmischung bezogen auf die Zusammensetzung.

In einer weiteren bevorzugten Ausführungsform kann die Wirkung der erfindungsgemäßen Wirkstoffkombination durch Emulgatoren gesteigert werden. Solche Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zu Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3 bis 6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüsts eine Hydroxylgruppe tragen und sowohl aus tierischem Gewebe (Zoosterine) wie auch aus pflanzlichen Fetten (Phytosterine) isoliert werden. Beispiele für Zoosterine sind das Cholersterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospholipide, die z.B. als Lecithine bzw. Phosphatidylcholine aus z.B. Eidotter oder Pflanzensamen (z.B. Sojabohnen) gewonnen werden, verstanden.
- Fettsäureestervon Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®}PGPH),
- Lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide, wie beispielsweise Glucose, Galactose, Fructose, Fruchtzucker und Lactose,
- Farbstoffe zum Anfärben des Mittels,
- Wirkstoffe wie Allantoin und Bisabolol, Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Pigmente,
- Treibmittel wie Propan-Butan-Gemische, N₂O, Dimethylether, CO₂ und Luft,
- Antioxidantien.

Die erfindungsgemäßen kosmetischen Reinigungsmittel unterliegen hinsichtlich ihrer Konfektionierungsform keinerlei Beschränkungen und können als Emulsion, Creme, Lösung, Gel oder Mousse formuliert werden.

Bevorzugte Ausführungsformen des erfindungsgemäßen kosmetischen Reinigungsmittels sind Shampoos, Duschbäder, Duschgele, Haarspülungen, Haarkuren, Rasierwässer und/oder Deodorantien.

Gemäß einer weiteren, besonders bevorzugten Ausführungsform der Erfindung wird das Reinigungsmittel als flüssiges Textilreinigungsmittel und/oder als flüssiges Waschmittel mit einen pH-Wert im Bereich von 4 bis 10, insbesondere von 5,5 bis 8,5 konfektioniert.

Es kann aber auch erfindungsgemäß bevorzugt sein, dass das Reinigungsmittel als Weichspüler mit einem pH-Wert im Bereich von 1 bis 6, insbesondere von 1,5 bis 3,5, formuliert wird.

In einer weiteren, besonders bevorzugten Ausführungsform der Erfindung wird das erfindungsgemäße Reinigungsmittel als flüssiges Reinigungsmittel für harte Oberflächen konfektioniert. Insbesondere bevorzugt innerhalb dieser Ausführungsform sind erfindungsgemäße Mittel, die als Bad- und/oder WC-Reiniger vorliegen und einen pH-Wert im Bereich von 1 bis 6, insbesondere von 1,5 bis 3,5 aufweisen, Allzweckreinigungsmittel, die einen pH-Wert von 4 bis 11, insbesondere von 6 bis 10 aufweisen sowie Handgeschirrspülmittel, die einen pH-Wert im Bereich von 4 bis 8, insbesondere von 5 bis 7 aufweisen.

Ein zweiter Gegenstand der Erfindung die Verwendung eines erfindungsgemäßen kosmetischen Mittels zur Verbesserung der sensorischen Eigenschaften der Haare.

Ein dritter Gegenstand der Erfindung ist die Verwendung eines erfindungsgemäßen kosmetischen Mittels zur innerstrukturellen Stabilisierung der Haare.

Ein vierter Gegenstand der Erfindung ist ein Verfahren zur Reinigung und Konditionierung von Haut und/oder Haaren, bei dem ein erfindungsgemäßes kosmetisches Mittel auf die Haut oder die Haare aufgetragen, und je nach Applikationsform nach einer Einwirkungszeit von 30 Sekunden bis zu mehreren Stunden wieder ausgespült wird.

Die folgenden Beispiele sollen die Erfindung näher erläutern, ohne sie darauf zu beschränken. Die aufgeführten Mengenangaben in den Beispielen beziehen sich, sofern nicht anders angegeben, auf Gew.-%.

### Beispiele:

### Pflegeshampoo 1:

| | |
|---|---|
| Texapon^{®1}K 14 S | 50 |
| Dehyton^{®2}K | 10 |
| Plantacare^{®3} 818 UP | 4 |
| D-Panthenol | 0,5 |
| Nicotinsäureamid | 0,3 |
| Terpolymer gem. Erfindung | 0,3 |
| Zitronensäure | 0,5 |
| Arlypon^{®4}F | 1,2 |
| NaCl | 1,3 |
| Konservierungsmittel | q.s. |
| Wasser | ad 100 |
| pH-Wert | 5 |

### Pflegeshampoo 2:

| | |
|---|---|
| Texapon^{®5}NSO | 40 |
| Dehyton^{®6}G | 6 |
| Terpolymer gem. Erfindung | 1,0 |
| Lamesoft^{®7} PO 65 | 4 |
| Gluadin^{®8}WQT | 2,5 |
| Gluadin^{®9}W 20 | 0,5 |
| D-Panthenol | 0,3 |
| Pantolacton | 1,0 |
| Zitronensäure | 0,5 |
| Na-benzoat | 0,5 |
| Parfüm | 0,4 |
| NaCl | 1,3 |
| Polyquaternium-10 | 0,2 |
| Wasser | ad 100 |
| pH-Wert | 5 |

### Weichspüler:

### (3-fach-Konzentrat und Regular)

| | **1** | **2** |
|---|---|---|
| Esterquat* | 15 | 5 |
| 2-Propanol | 1,7 | 0,55 |
| MgCl₂ | 0,1 | -- |
| Terpolymer gem. Erfindung | 0,8 | 1,2 |
| Farbstoff | + | + |
| Parfüm | + | + |
| Wasser | Ad 100 | Ad 100 |

| | | |
|---|---|---|
| * N-Methyl-N-(2-hydroxyethyl)-N,N-(ditalgacyloxyethyl)ammonium-methosulfat | | |

### Flüssigwaschmittel:

| | **Gew.-%** |
|---|---|
| C₁₂₋₁₈-Fettsäure, Na-Salz | 7 |
| Polyacrylat-Verdicker | 1,5 |
| C₁₂₋₁₈-Fettalkohol mit 7 EO | 12 |
| Natriumlaurylethersulfat mit 2 EO, Na-Salz | 5 |
| Lineare C₁₀-C₁₃-Alkylbenzolsulfonsäure, Na-Salz | 10 |
| Citronensäure, Na-Salz | 2,5 |
| Phosphonsäure, Na-Salz | 1 |
| Borsäure, Na-Salz | 1 |
| Optischer Aufheller | 0,08 |
| 1,2-Propandiol | 5 |
| Silikon-Entschäumer | 0,15 |
| Cellulase | 0,08 |
| Protease | 1,2 |
| Amylase | 0,5 |
| Parfüm | 1,5 |
| Terpolymer gem. Erfindung | 1,0 |
| Wasser | Ad 100 |

### Verzeichnis der eingesetzten Handelsprodukte:

- 1: INCI-Bezeichnung: Sodium Myreth Sulfate; AS: etwa 27%; Cognis
- 2: INCI-Bezeichnung: Cocamidopropylbetaine; AS: etwa 30%; Cognis
- 3: INCI-Bezeichnung: Coco Glucoside; AS: etwa 51-53%; Cognis
- 4: INCI-Bezeichnung: Laureth-2; Cognis
- 5: INCI-Bezeichnung: Sodium Laureth Sulfate; AS: etwa 28%; Cognis
- 6: INCI-Bezeichnung: Disodium Cocoamphodiacetate; AS: etwa 30%; Cognis
- 7: INCI-Bezeichnung: Coco Glucoside, Glyceryl Oleate; AS: etwa 65%; Cognis
- 8: Weizenproteinhydrolysat; Cognis
- 9: INCI-Bezeichnung: Hydrolized Wheat Protein; Cognis

## Patentansprüche

1. Tensidhaltiges Reinigungsmittel, enthaltend - bezogen auf sein Gesamtgewicht - 0,001 bis 10 Gew.-% mindestens eines kationischen Terpolymers, das zusammengesetzt ist aus
a) mindestens einem Vinylimidazol-Monomeren,
b) mindestens einem Vinyl-Pyrrolidon-Monomeren und
c) Poly(dimethyldiallylammoniumchlorid),
**dadurch gekennzeichnet, dass** das Mittel einen pH-Wert im Bereich von 2 bis 5,5 aufweist, wenn es als kosmetisches Reinigungsmittel dient, und einen pH-Wert im Bereich von 2 bis 11, wenn es als Reinigungsmittel für harte Oberflächen und/oder Textilien formuliert wird.

2. Mittel nach Anspruch 1, **dadurch gekennzeichnet, dass** die kationische Ladungsdichte des Terpolymers 2,5 bis 5 meq/g, bevorzugt 3 bis 4,5 meq/g und insbesondere 3,5 bis 4 meq/g (bei pH 7), und das Molekulargewicht 50,000 bis 500,000, bevorzugt 75,000 bis 300,000 und insbesondere 100,000 bis 200,000 beträgt.

3. Mittel nach einem der Ansprüche 1 oder 2, **dadurch gekennzeichnet, dass** es mindestens ein anionisches Tensid sowie mindestens ein weiteres Tensid, ausgewählt aus der Gruppe der amphoteren/zwitterionischen und der nichtionischen Tenside enthält.

4. Kosmetisches Mittel nach einem der Ansprüche 1 bis 3, **dadurch gekennzeichnet, dass** es ein Gemisch aus anionischen Tensiden mit amphoteren/zwitterionischen und/oder nichtionischen Tensiden enthält, wobei das Gewichtsverhältnis des anionischen Tensids zu den amphoteren/zwitterionischen und nichtionischen Tensiden (1-2) : (0,5-1) : (0,5-1) beträgt.

5. Kosmetisches Mittel nach einem der Ansprüche 1 bis 4, **dadurch gekennzeichnet, dass** es weiterhin Panthenol enthält sowie mindestens einen weiteren Haarpflegewirkstoff, der ausgewählt ist aus der Gruppe der Vitamine und/oder Provitamine, der Proteinhydrolysate, der natürlichen oder synthetischen Ölkomponenten und/oder der Pflanzenextrakte.

6. Kosmetisches Mittel nach einem der Ansprüche 1 bis 5, **dadurch gekennzeichnet, dass** es mindestens einen Antischuppenwirkstoff enthält.

7. Kosmetisches Mittel nach einem der Ansprüche 1 bis 6, **dadurch gekennzeichnet, dass** es weiterhin mindestens einen Vertreter aus der Gruppe der kationischen Tenside, der kationischen Polymere, die sich von dem Terpolymer unterscheiden, der UV-Filter, der Aminosäuren der wasserunlöslichen Silikone, der wasserlöslichen Silikone und/oder der Amodimethicone
enthält.

8. Mittel nach einem der Ansprüche 1 bis 3, **dadurch gekennzeichnet, dass** es als flüssiges Textilreinigungsmittel vorliegt und im Falle eines flüssigen Waschmittels einen pH-Wert im Bereich von 4 bis 10, insbesondere von 5,5 bis 8,5 sowie im Falle eines Weichspülers einen pH-Wert im Bereich von 1 bis 6, insbesondere von 1,5 bis 3,5, aufweist.

9. Mittel nach einem der Ansprüche 1 bis 3, **dadurch gekennzeichnet, dass** es als flüssiges Reinigungsmittel für harte Oberflächen vorliegt und im Falle eines Bad- und/oder WC-Reinigers einen einen pH-Wert im Bereich von 1 bis 6, insbesondere von 1,5 bis 3,5, im Falle eines Allzweckreinigungsmittels einen pH-Wert von 4 bis 11, insbesondere von 6 bis 10 sowie im Falle eines eines Handgeschirrspülmittels einen pH-Wert im Bereich von 4 bis 8, insbesondere von 5 bis 7 aufweist.

10. Verwendung eines Mittels nach einem der Ansprüche 1 bis 7 zur Verbesserung der sensorischen Eigenschaften der Haare.

11. Verwendung eines Mittels nach einem der Ansprüche 1 bis 7 zur innerstrukturellen Stabilisierung der Haare.

12. Verfahren zur Reinigung und Konditionierung von Haut und/oder Haaren, **dadurch gekennzeichnet, dass** ein Mittel nach einem der vorhergehenden Ansprüche auf die Haut oder die Haare aufgetragen, und je nach Applikationsform nach einer Einwirkungszeit von 30 Sekunden bis zu mehreren Stunden wieder ausgespült wird.
